# EUROPEAN PATENT SPECIFICATION

(11) **EP 3 218 019 B1**
(45) Date of publication and mention of the grant of the patent: **17.02.2021**
(21) Application number: 15858646.1
(22) Date of filing: 13.11.2015
(51) Int. Cl.: A61L 27/02, A61L 27/44, A61L 27/46, A61F 2/28, B02C 1/00, A61L 27/56, B02C 17/18, B29C 64/00, A61K 9/00, A61K 33/00, A61K 47/34, B33Y 80/00

(54) **BIORESORBABLE-MAGNESIUM COMPOSITE**
BIORESORBIERBARER MAGNESIUMVERBUNDSTOFF
COMPOSITE DE MAGNÉSIUM BIORÉSORBABLE

(30) Priority: 14.11.2014 SG 10201407605R
(43) Date of publication of application: 20.09.2017
(73) Proprietor: Nanyang Technological University, Singapore 639798 (SG)
(72) Inventor: TEOH, Swee-hin, Singapore 639798 (SG); LIM, Jing, Singapore 639798 (SG); CHONG, Mark Seow Khoon, Singapore 639798 (SG)
(74) Representative: Mewburn Ellis LLP
(86) International application number: PCT/SG2015/050449
(87) International publication number: WO 2016/076800

(56) References cited:
- EP-A1- 1 742 680
- EP-A1- 2 749 301
- WO-A1-2008/040763
- WO-A2-2008/117043
- WO-A2-2014/123978
- US-A- 5 899 939
- US-B2- 6 921 544
- US-B2- 8 501 056
- KWON B. K. ET AL.: 'Magnesium Chloride in a Polyethylene Glycol Formulation as a Neuroprotective Therapy for Acute Spinal Cord Injury: Preclinical Refinement and Optimization.' J. NEUROTRAUMA vol. 26, no. 8, 03 August 2009, pages 1379 - 1393, XP008167305
- ZHOU H. ET AL.: 'Fabrication of Novel Poly(lactic acid)/Amorphous Magnesium Phosphate Bionanocomposite Fibers for Tissue Engineering Applications via Electrospinning.' MATERIALS SCIENCE AND ENGINEERING C vol. 33, 31 January 2013, pages 2302 - 2310, XP029002410
- LIM J. ET AL.: 'Polymer Powder Processing of Cryomilled Polycaprolactone for Solvent-Free Generation of Homogeneous Bioactive Tissue Engineering.' SMALL vol. 10, 17 April 2014, pages 2495 - 2502, XP009502817

## Description

### CROSS-REFERENCE TO RELATED APPLICATION

This application claims the benefit of priority of Singapore Patent Application No. 10201407605R, filed November 14, 2014.

### TECHNICAL FIELD

The disclosure relates generally to magnesium biocomposites. More specifically, the use of elemental magnesium or magnesium alloy in the biocomposite is minimized and preferably avoided. The magnesium biocomposites can be used in the field of orthopaedic.

### BACKGROUND

Magnesium (Mg) is an essential trace element of the human body, and has been shown to play an important role in regulating biological functions, including that of bone homeostasis. Currently, Mg has been administered as a dietary supplement, to be taken orally to regulate bone mass and maintain bone health. Many patients who suffer from poor bone mass and those who are pre-disposed to arthritis have been put on Mg-rich diet due to the fact that Mg is important for bone mineralization. In addition, medical practitioners employ the use of Mg for improving calcium (Ca) uptake, particularly in cases where an already-present, exogenous supply of calcium is ineffective. In the case of orthopaedic implants, recent developments have seen the use of Mg-coated implants for better host-implant integration.

Current systems employ the use of magnesium as an alloy in orthopaedic implants. These implants are faced with challenges in controlling its degradation in vivo, due to the potential side-effects of locally produced gas near its surface

In WO 2008/040763 a cryomilling method is disclosed which allows for the formation of an implant comprising a polymeric matrix and a magnesium salt.

Accordingly, there remains a need to provide for an alternative magnesium composition that overcomes, or at least alleviates, the above problem.

### SUMMARY

The present disclosure makes use of low temperature pulverization of materials for forming biomaterials or biocomposites suitable for delivering magnesium to a subject to facilitate bone growth and repair, regeneration, and/or proliferation of host tissues. In particular, the biocomposite of present disclosure includes a polymeric matrix and a magnesium filler. The polymeric matrix may be provided for by any suitable biocompatible and/or biodegradable polymer (including copolymer). The magnesium filler may be provided for by any suitable soluble magnesium salt.

The present disclosure also provides for a method for forming the present biocomposite. The method includes low temperature processing of the biocompatible and/or biodegradable polymer (including copolymer) and the magnesium filler to form powders. The method further includes processing of the powders to form a thin film or a three-dimensional scaffold of the biocomposite.

The present disclosure further provides for said biocomposites for use in a method for promoting bone growth and repair, regeneration, and/or proliferation of host tissues. The method includes implanting into a subject the present biocomposite at a site in need of bone growth and repair, regeneration, and/or proliferation of host tissues The invention is set out in the appended set of claims.

### BRIEF DESCRIPTION OF THE DRAWINGS

In the drawings, like reference characters generally refer to the same parts throughout the different views. The drawings are not necessarily drawn to scale, emphasis instead generally being placed upon illustrating the principles of various embodiments. In the following description, various embodiments of the disclosure are described with reference to the following drawings.

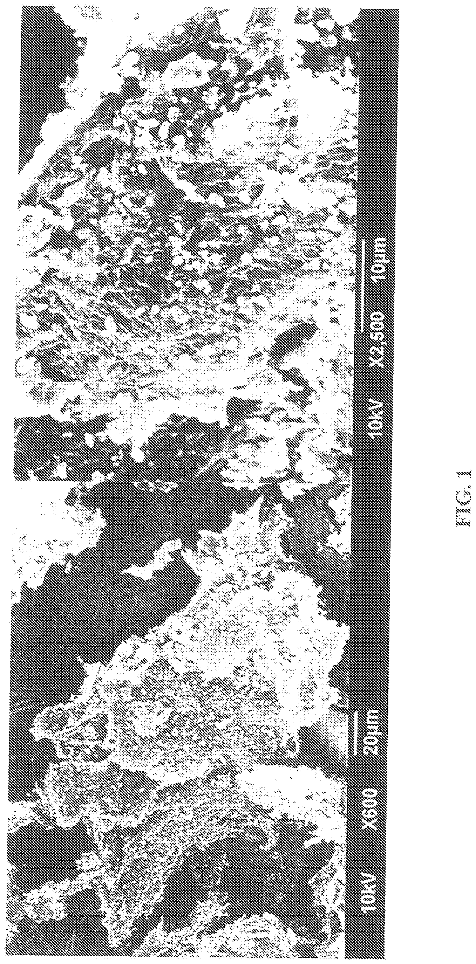

FIG. 1 shows scanning electron microscopy images of cryomilled polycaprolactone (PCL)/tricalcium phosphate powders according to Example 1, at 600x and 2500x magnification, demonstrating that homogenous distribution of tricalcium phosphate (granular, approximately 2 µm) was achieved.

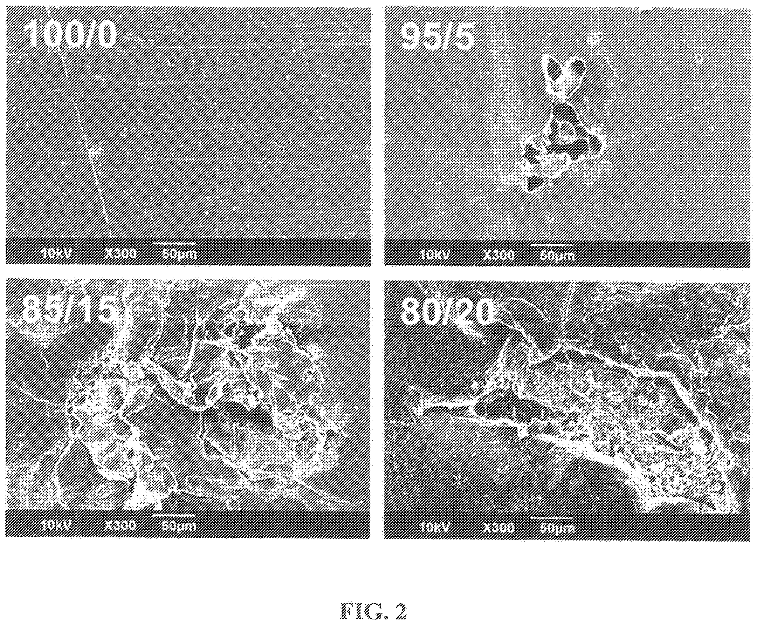

FIG. 2 shows representative scanning electron microscopy (SEM) images of various PCL/Mg films after immersing in phosphate buffer solution (PBS) at 37 °C for 4 hours according to Example 2. 100/0 represents PCL (100 wt%) without incorporated Mg (0 wt%). As the amount of Mg increases (i.e. 95/5, 85/15, 80/20 PCL/Mg), the size and the number of pores increased. Images were taken at 300x magnification, and scale bar represents 50 microns.

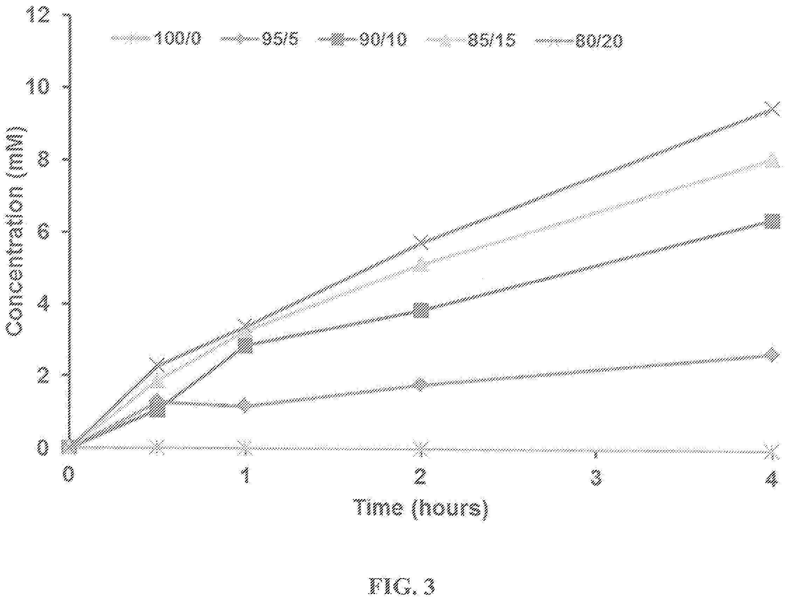

FIG. 3 shows the release profiles of various PCL/Mg films over 4 hours. 100/0 (i.e. pure PCL) did not exhibit any release while increasing amounts of Mg led to increased release, and higher rates of release.

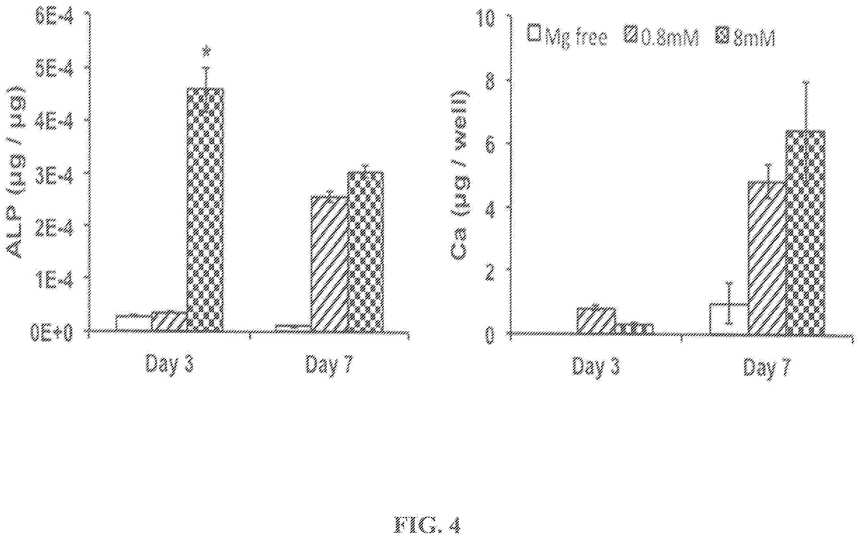

FIG. 4 shows alkaline phosphatase (ALP) activity and Ca deposition of mesenchymal stem cells (MSCs) cultured in the absence of Mg (Mg free), normal serum (0.8 mM), and elevated Mg (8 mM) according to Example 4. Results indicated a peak in ALP activity on Day 3 in the 8 mM group, while showing 9 times higher activity as compared to Mg free and 0.8 mM groups. Ca deposition was markedly higher on Day 7 in the 8 mM group.

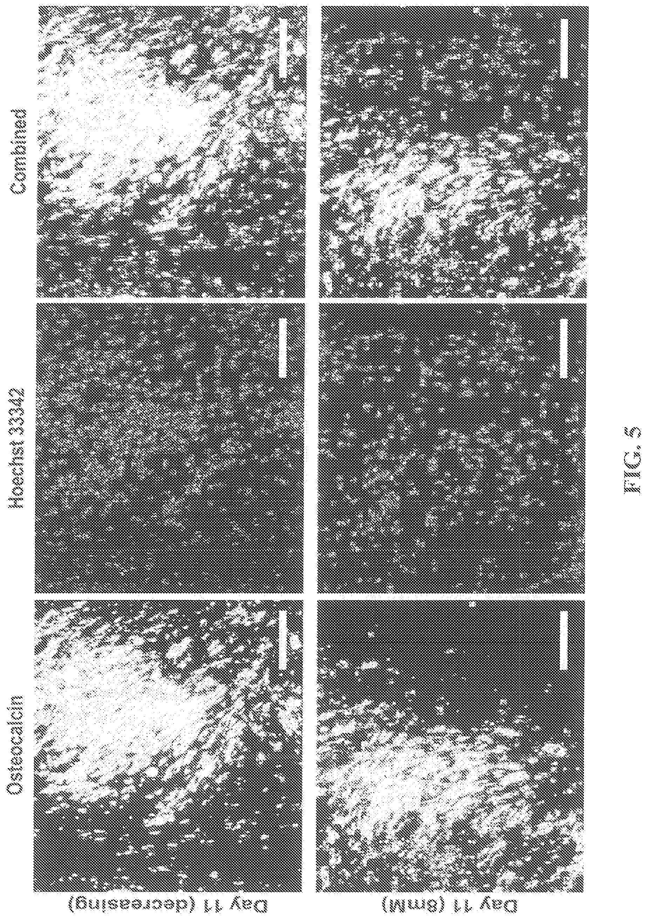

FIG. 5 shows osteocalcin expression of MSCs cultured on Day 11 according to Example 4, demonstrating the ability of maintaining osteogenic behaviour in the presence of long, prolonged exposure to elevated levels of Mg, as compared to MSCs cultured in initially high levels of Mg and slowly decreased to normal serum levels (0.8 mM). Images were taken at 4x magnification, and the scale bar represents 600 µm.

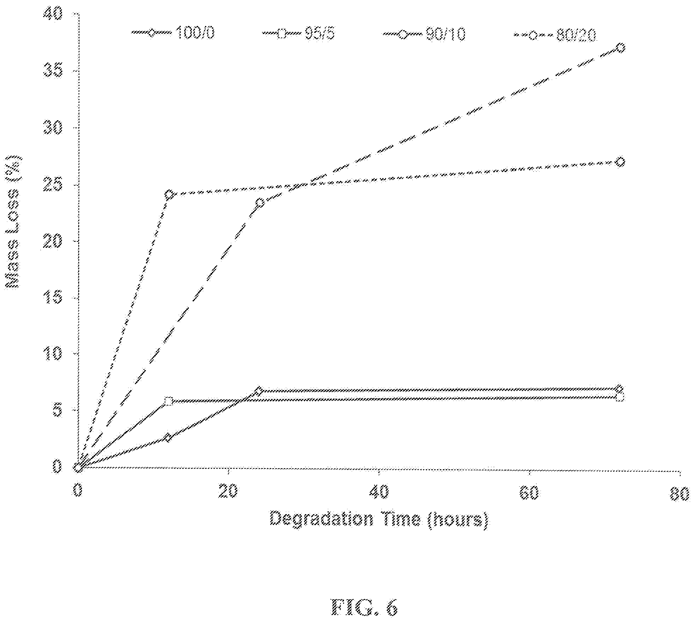

FIG. 6 shows mass loss profiles of various PCL/Mg biocomposite films according to Example 5. 100/0 and 95/5 PCL/Mg films behaved similarly, showing minimal mass loss (approximately 5 %) over the first 72 hours. On the other hand, 90/10 and 80/20 PCL/Mg films showed increased mass losses, and attained at least 25 % mass loss within the same time frame.

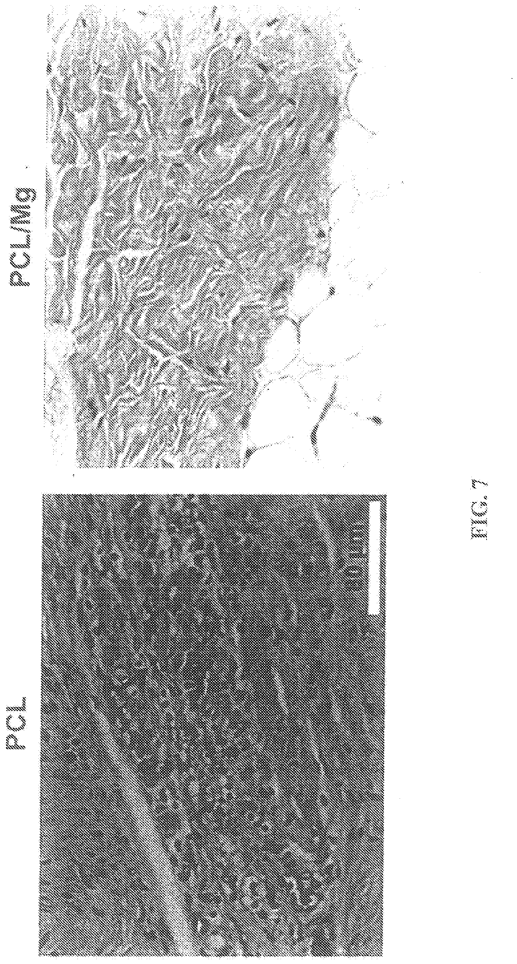

FIG. 7 shows hematoxylin and eosin (H&E) stains of PCL and PCL/Mg films implanted into the fatty pockets of pigs over a period of 3 months according to Example 6. Darkly stained cell nuclei, indicative of inflammatory events, were seen in the tissue structures surrounding the PCL films, while minimal indications of inflammation were observed in the PCL/Mg films.

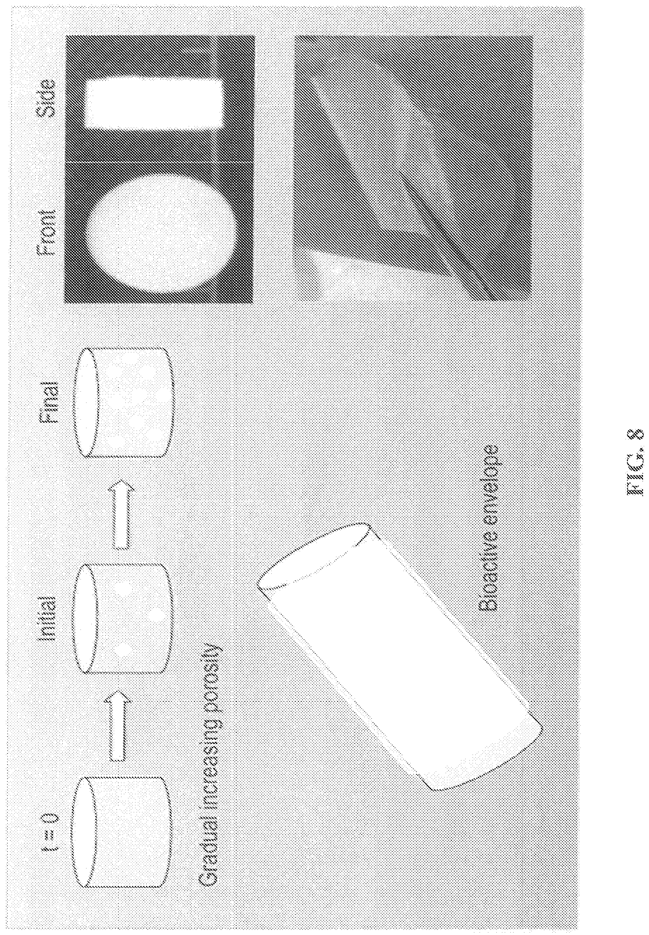

FIG. 8 shows an illustration and prototype of a 3D scaffold with gradually increasing porosity, and a bioactive thin film that may be used as an envelope to guide bone tissue regeneration according to Example 7.

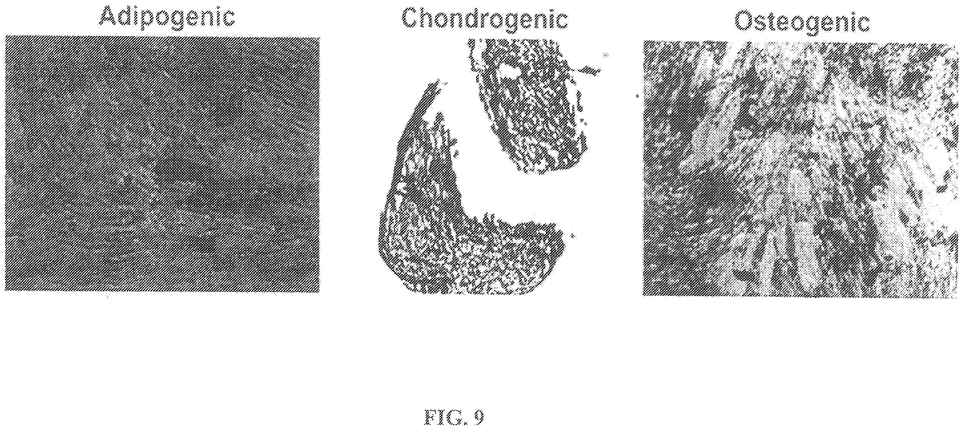

FIG. 9 shows the differentiation of human fetal mesenchymal stem cells (hfMSCs) into the following three lineages: adipogenic, chondrogenic, and osteogenic according to Example 8.

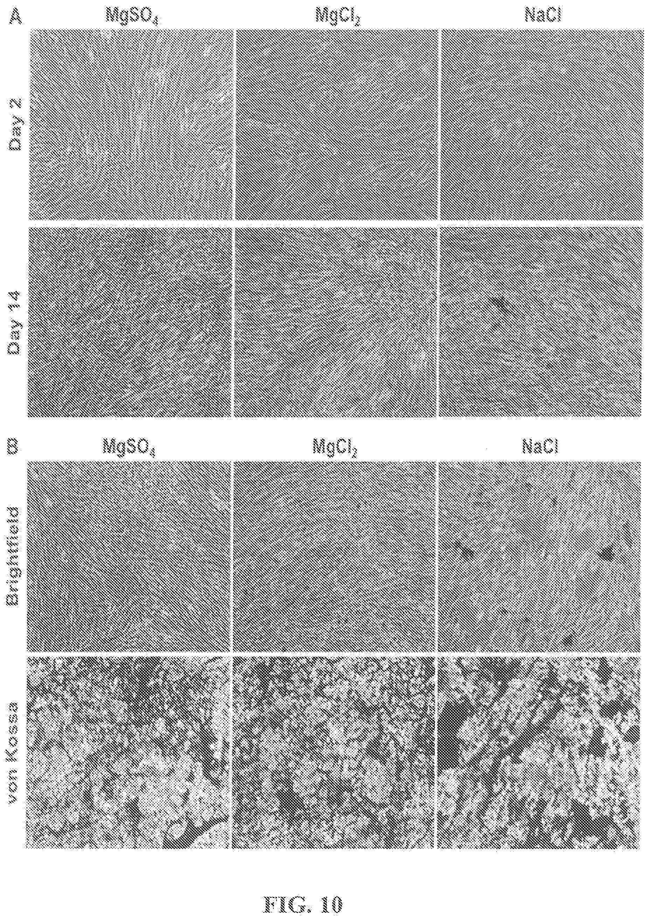

FIG. 10 shows the results of proliferation and differentiation of hfMSCs enabled by both magnesium chloride (MgCl₂) and magnesium sulphate (MgSO₄) according to Example 8. NaCl was used as a control to demonstrate that Cl⁻ did not influence proliferation and differentiation events.

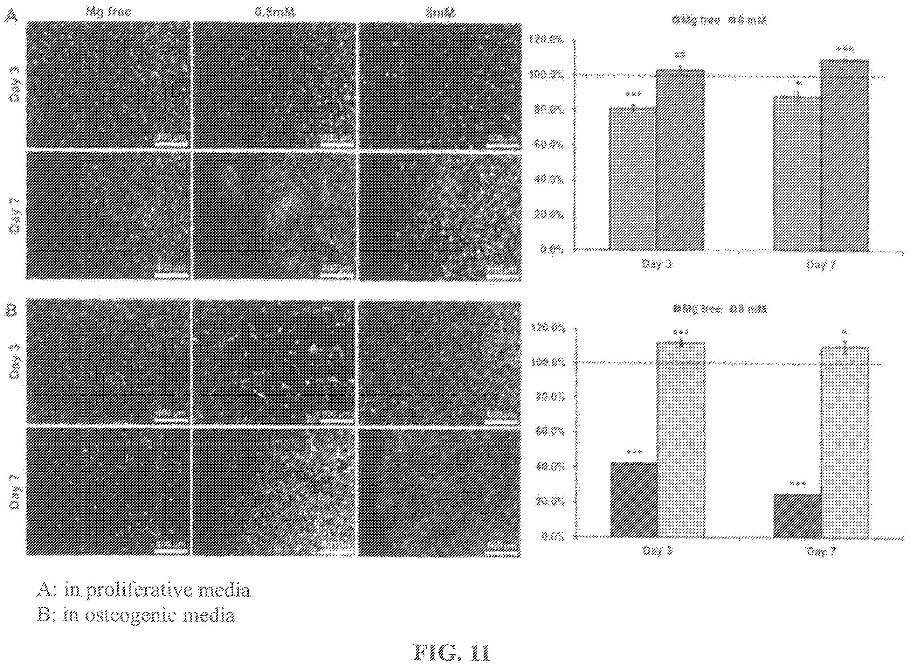

FIG. 11 shows the effect of various Mg levels on hfMSC proliferation. hfMSC proliferation in the Mg free and 8 mM groups were compared and normalized against the basal level (0.8 mM), in both (A) proliferative and (B) osteogenic media. In both proliferative and osteogenic media, Mg starvation suppressed cell growth to a particularly large extent (p < 0.001). On the other hand, 8 mM of Mg supported cell proliferation (p < 0.001). Corresponding visualization with live/dead (FDA/PI) imaging led to corroborating results, with higher Mg indicating higher hfMSC proliferation.

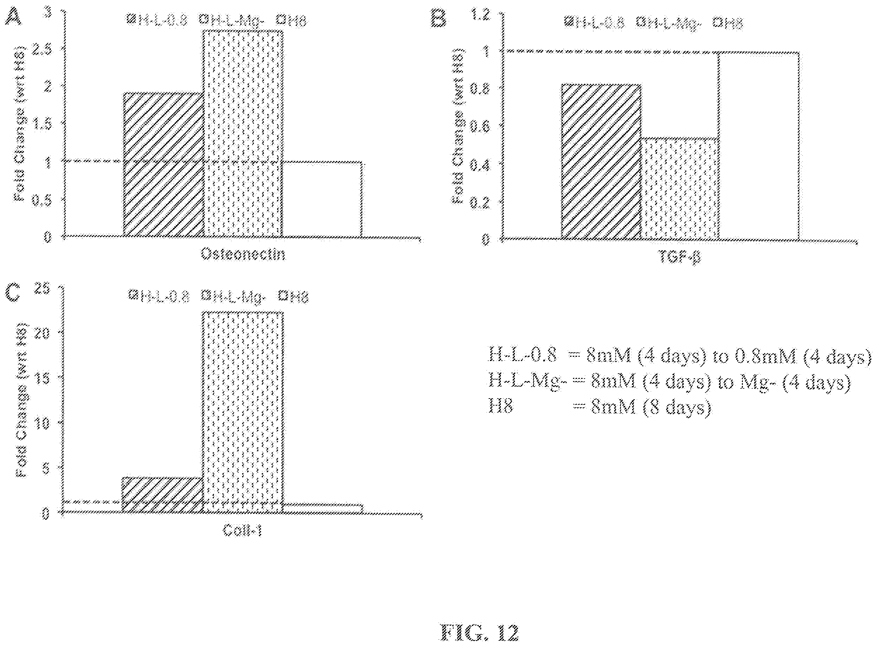

FIG. 12 shows the effect of Mg on osteogenic differentiation according to Example 8. hfMSCs cultured under prolonged exposure to high levels of Mg (8 mM) exhibited lower levels of osteonectin (ON), collagen type I (coll-I), and transforming growth factor*-beta* (TGF-β) expressions (FIG. 12). Upon switching to Mg-free conditions after 4 days, hfMSCs demonstrated higher potential for osteogenic differentiation as compared to 0.8 mM.

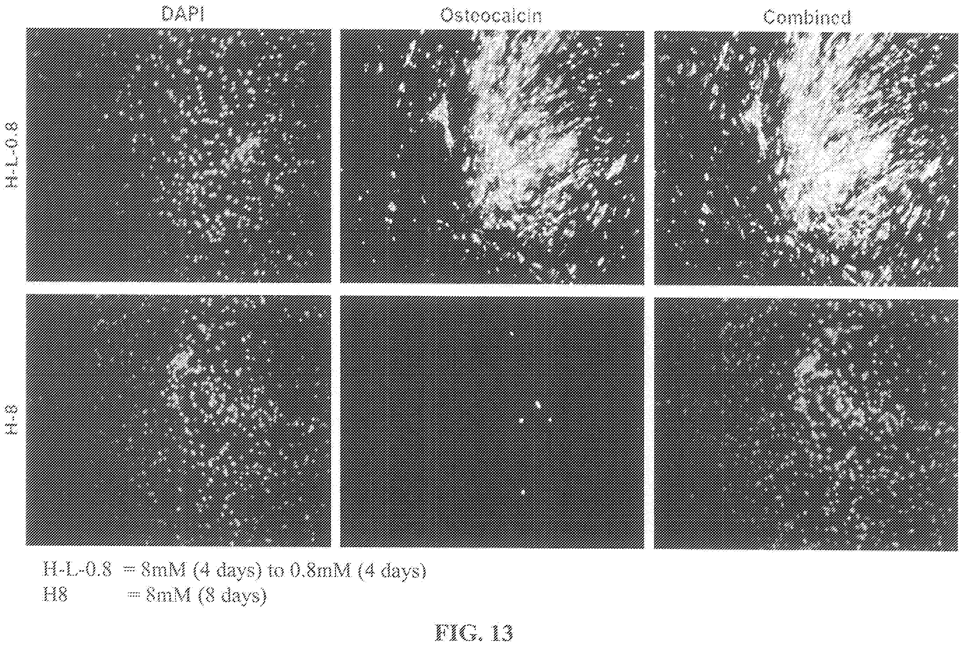

FIG. 13 shows osteocalcin (OC) protein expression as determined using immunocytochemical staining. From the results, OC expression was clearly demonstrated in the group exposed to decreasing concentrations of Mg, while prolonged exposure to Mg resulted in suppressed expression of OC from the hfMSCs.

### DESCRIPTION

The following detailed description refers to the accompanying drawings that show, by way of illustration, specific details and embodiments in which the disclosure may be practised. These embodiments are described in sufficient detail to enable those skilled in the art to practise the disclosure. The various embodiments are not necessarily mutually exclusive, as some embodiments can be combined with one or more other embodiments to form new embodiments.

The present disclosure discloses a fabrication method of a biocomposite comprising a polymeric matrix and a magnesium filler via a solvent-free and a heat-free technique. In other words, the formation technique does not involve a solvent. The formation technique further does not involve a heating step.

The polymeric matrix is preferably a well-studied biomaterial that is approved for use in clinics by the Food and Drug Administration (FDA) of the United States. In one example, polycaprolactone (PCL) has been used as a long-term drug delivery device, and has been employed as scaffolds for tissue engineered bone and cartilage, and more recently, for bone repair. The biomaterial preferably has a long degradation time.

Other suitable biomaterials include, but not limited to, poly(lactic-co-glycolic acid) (PLGA), poly(lactic acid) (PLA), poly(glycolic acid) (PGA), the family of polyhydroxyalkanoates (PHA), polyethylene glycol (PEG), polypropylene glycol (PPG), polyesteramide (PEA), poly(lactic acid-co-caprolactone), poly(lactide-co-trimethylene carbonate), poly(sebacic acid-co-ricinoleic acid) and a combination thereof. The polymeric matrix may include one or more of the biomaterials.

Magnesium salts are chosen as the filler material due to their role in maintaining normal cellular function, and more specifically, for their role in regulating bone homeostasis. Recent studies have shown that osteogenic activities are regulated by Mg. According to the invention , a soluble magnesium salt as the filler is incorporated into the polymeric matrix. Suitable magnesium salts are those that dissolve in an aqueous environment or medium, and include, but not limited to, magnesium chloride (MgCl₂), magnesium sulphate (MgSO₄), or magnesium phosphate (Mg₃(PO₄)₂).

Importantly, the use of elemental magnesium or magnesium alloy in the biocomposite is minimized and preferably avoided. According to the invention, the biocomposite is rendered porous if the biocomposite is initially non-porous, or rendered more porous if the biocomposite is initially porous, by leaching or dissolving the magnesium salt upon contact with an aqueous environment or medium. This finds particular use as implants or scaffolds where the biocomposite affords the ability to create a gradually porous scaffold over time, matched by the simultaneous dissolution of Mg into the surrounding microenvironment after implantation into the body of a subject. In this sense, the gradual increasing porosity of a biocomposite scaffold is symbolic of a 'smart' scaffold.

Another advantage of the biocomposite lies in the release of Mg, which has been demonstrated and established to be an important trace element for potentiating osteogenic differentiation.

As illustrated in the examples described in later paragraphs, the amount of magnesium filler initially present in the biocomposite may affect the degradation time of the polymeric matrix and cellular response to the magnesium. For example, based on the examples results it is hypothesized that early supplementation of Mg directs osteogenic differentiation of mesenchymal stem cells (MSCs). By exposing MSCs to elevated levels of Mg (8 mM) for four days and subsequently switching back to basal (0.8 mM) and Mg-free conditions, it was demonstrated that osteogenic factors such as ALP, osteonectin (ON), collagen-type 1 (coll-1) were upregulated, as compared to prolonged exposure of elevated Mg levels. Taken together, these results suggest that extracellular Mg may play important roles in bone tissue engineering. According to the invention, the magnesium filler is present in 10 wt%, 11 wt%, 12 wt%, 13 wt%, 14 wt%, 15 wt%, 16 wt%, 17 wt%, 18 wt%, 19 wt%, or 20 wt% based on the total weight of the biocomposite.

As mentioned in earlier paragraphs, the biocomposite fabrication technique does not involve a solvent or a heating step. According to the invention, the method for forming the present biocomposite includes first mixing of a polymeric matrix and a magnesium filler. After mixing, the mixture is processed in a cryomill to obtain fine powder. The composites were pre-weighed using a microbalance and loaded into a cryogenic vial with a ball-to-mass ratio of 30:1. The cryomilling protocol was set to be 6 to 8 min of pre-cooling in liquid nitrogen and 20 min of continuous milling for one cycle. One advantage of employing a cryomilling technique is that particle size reduction efficiency is improved and homogenous distribution may be achieved in a single processing step.

The fine powder is further processed to form a thin film or a three-dimensional (3D) scaffold. In certain embodiments, the 3D scaffold may be fabricated using an additive manufacturing technique or using a die set along with the incorporation of 50 vol% of sodium chloride, followed by leaching in water.

In one embodiment, a thin film, such as 60 microns or less in thickness, of the biocomposite can be formed by pressing the fine powder between two stainless steel sheets. For example, the PCL composite films may be thermally pressed into films of thickness approximately 30 to 60 µm. Briefly, a known mass of composite is placed between two stainless steel sheets on a heat press system with temperature control. Temperature is elevated to 100 °C and pressure is applied for 30 min. The pressed film is then allowed to cool to room temperature via normal convection cooling.

The continuity of the biocomposite thin film structure has been shown to play a considerable role in bone and vascular regeneration, both of which are important in tissue regeneration. The thin film fabricated by the present method is preferably a continuous film and is non-porous. While a porous thin film may be desired for directing ingrowth, a significant trade-off is present in the mechanical properties of the porous biocomposite, as a substantially porous material may have compromised mechanical properties. Present biocomposite provides an important advantage in that the biocomposite can be made substantially non-porous at the beginning to provide better mechanical integrity. Subsequently, upon interaction *in vivo* with body fluid, soluble magnesium will be leached out over time, gradually creating a porous structure that may bear resemblance to other existing films and/or scaffolds.

Accordingly, the present disclosure further provides said biocomposites for use in a method for promoting bone growth and repair, regeneration, and/or proliferation of host tissues. The method includes implanting into a subject the present biocomposite at a site in need of bone growth and repair, regeneration, and/or proliferation of host tissues.

In order that the disclosure may be readily understood and put into practical effect, particular embodiments will now be described by way of the following non-limiting examples.

### EXAMPLES

### EXAMPLE 1

Cryomilling was employed as a method to achieve efficient and homogenous distribution of presently disclosed biocomposite. Polycaprolactone (PCL) particles were pulverized into fine powder after a cryomilling process of 20 min. It is shown in the high magnification images of FIG. 1 that the filler (in this illustration, tricalcium phosphate) was well distributed in the PCL matrix.

### EXAMPLE 2

To demonstrate an increased surface porosity of presently disclosed biocomposites, a biocomposite including PCL and a soluble magnesium salt (i.e. a PCL/Mg biocomposite) was processed by cryomilling, and subsequently fabricated into a continuous film structure (FIG. 2). These biocomposite films were then soaked in a phosphate buffer solution (PBS) at 37 °C. PBS is a buffer solution commonly used to simulate bodily fluids. After immersing for various times up to 4 hours, the films were then retrieved and imaged, revealing a highly porous surface due to the selective leaching of Mg. The porosity of the biocomposite films increased with increasing amounts of Mg salt.

### EXAMPLE 3

To demonstrate the release profile of Mg from presently disclosed biocomposites, samples were taken from the PBS in which the PCL/Mg films were immersed according to Example 2. The results are presented in FIG. 3. As expected, 100/0 (i.e. pure PCL) films did not exhibit any release of Mg into PBS over the release period. Mg release correlated directly with the amount of Mg incorporated, increasing with 80/20 PCL/Mg demonstrating the highest release at the end of 4 hours. In addition, the rate of release also increased with increasing amount of Mg.

### EXAMPLE 4

To demonstrate that Mg is able to promote osteogenic differentiation, *in vitro,* cellular studies were conducted using mesenchymal stem cells (MSCs) in the presence of elevated levels of Mg. MSCs are known to be present within the bone marrow niche and play a role in regulating bone by differentiating into osteoblastic or osteoclastic phenotypes. From the results, alkaline phosphatase (ALP) activity peaked at Day 3 in the presence of 8 mM of Mg, showing 9 times higher expression levels as compared to MSCs cultured in the absence of Mg (Mg free) and under normal serum conditions (0.8 mM). Calcium deposition was found to be markedly higher on Day 7 as compared to both Mg free and 0.8 mM groups (FIG. 4). Immunocytochemical (ICC) staining of MSCs with osteocalcin demonstrated expression at Day 11 (FIG. 5), regardless of the exposure time to elevated levels of Mg.

### EXAMPLE 5

To demonstrate that presently disclosed biocomposites exhibit a reduced degradation time, PCL/Mg biocomposite films were placed in 1 M sodium hydroxide (NaOH) solution at 37 °C. Results were plotted out in terms of mass loss (%) against time (hours) (FIG. 6), and indicated that with increase in Mg incorporation into the PCL biocomposites, degradation was significantly accelerated particularly in the case of 90/10 and 80/20 biocomposite films, where mass loss reached at least 25 %. On the other hand, 95/5 films behaved in a similar fashion to pure PCL films, showing approximately 5% mass loss over the first 72 hours.

### EXAMPLE 6

Presently disclosed 80/20 PCL/Mg films were implanted into pigs over a period of three months. While both PCL and PCL/Mg biocomposite films were well-accepted by the host without events of rejection, the inflammatory response was markedly different (FIG. 7). Darkly-stained cell nuclei indicative of inflammatory events persisted at 3 months in the PCL group, while minimal inflammation was observed in the PCL/Mg group, suggesting that PCL/Mg biocomposite films have the advantage in regulating the inflammatory environment upon implantation.

### EXAMPLE 7

The presently disclosed PCL/Mg biocomposite may be fabricated as a 3-dimensional (3D) scaffold body for use as a supporting architecture for directing bone in-growth while providing mechanical stability during the regenerative process. Its gradually increasing porosity promotes bone tissue in-growth (FIG. 8) while the release of Mg stimulates osteogenic differentiation of MSCs. In this instance, the 3D scaffold can be used within the craniomaxillofacial area, under slight to moderate mechanical loading.

The PCL/Mg biocomposite may also be fabricated as a thin film to serve as a bioactive sheet that has good flexural properties and high strength for use as an envelope to prevent fibrous tissue invasion while promoting osteogenic growth to bridge the defect area (FIG. 8). For instance, the thin film may be of not more than 50 µm thick.

### EXAMPLE 8

In this example, the influence of exogenous magnesium on mesenchymal stem cell proliferation and early osteogenic activity is investigated. Highly osteogenic human fetal mesenchymal stem cells (hfMSCs) were cultured in varying concentrations of Mg and in varying exposure times to Mg in an attempt to study the effects of prolonged and transient exposures to elevated concentrations of Mg on hfMSC proliferation and osteogenesis. From the results, exposure to elevated levels of Mg (8 mM) led to improved proliferation of hfMSCs as compared to basal levels (0.8 mM), while prolonged exposure to Mg-free conditions resulted in significant cell death. When hfMSCs were cultured in 8 mM Mg for 4 days and subsequently maintained in lower Mg concentrations, osteonectin (ON), collagen-1, bone morphogenetic protein-1, -4, -6 (BMP-1, -4, -6) were significantly upregulated as compared to cultures maintained in prolonged, elevated levels of Mg over 8 days. Taken together, these results suggest that an initial elevated level of Mg is necessary to kick-start the osteogenic differentiation of MSCs.

### MATERIALS AND METHODS

### Human fetal MSCs isolation and culture

Human fetal MSCs (hfMSCs) were obtained as previously described (Zhang Z-Y, Teoh S-H, Chong MSK, Lee ESM, Tan L-G, Mattar CN, et al. Neo-vascularization and bone formation mediated by fetal mesenchymal stem cell tissue-engineered bone grafts in critical-size femoral defects. Biomaterials. 2010;31:608-20*).* Cells were seeded in a flask (T175, Nunc, Rochester) at a density of 10⁶/ml in Dulbecco's Modified Eagle's medium (DMEM) supplemented with 10 % fetal bovine serum (FBS) and 1 % penicillin/streptomycin (pen/strep) (D10). Non-adherent cells were removed with media change on day three. The remaining adherent cells were subsequently used for this work (Passage 3 - 6).

### Multilineage mesenchymal differentiation of hfMSCs

The multilineage differentiation potential of hfMSCs was evaluated for the following: adipogenic, chondrogenic, and osteogenic differentiation. To induce adipogenic differentiation, cells were plated and cultured in adipogenic media (basal D10 supplemented with 5 µg/ml insulin, 10⁻⁶ M dexamethasone and 0.6 x 10⁻⁴ indomethacin (Sigma Aldrich, USA) for 21 days with media change every three days. Oil-Red O staining was then conducted for the presence of lipid vacuoles. For the induction of chondogenic differentiation, hfMSCs were pelleted and cultured in chondrogenic media DMEM supplemented with 0.1 µM dexamethasone, 0.17 mM ascorbic acid, 1.0 mM sodium pyruvate, 0.35 mM L-Proline, 1 % ITS (BD Pharmingen, USA), 1.25 mg/ml BSA, 5.33 µg/ml linoleic acid, 0.01 µg/ml TGF-β) for 28 days with media change every three days. The pellets were fixed with formalin, embedded in paraffin wax and cut before staining with Safranin O. To induce osteogenic differentiation, hfMSCs were cultured in osteogenic media (D10 supplement with 10 mM β-glycerophosphate, 10⁻⁸ dexamethasone, 0.2 mM ascorbic acid) for 21 days, with media change every three days. The cells were then fixed in 4 % paraformaldehyde and stained with von Kossa (2 w/v% silver nitrate), and exposed to ultraviolet light for 30 mins.

### Experimental culture of hfMSCs

hfMSCs that were isolated were exposed to the following conditions: For this purpose, Mg-free DMEM (BioRev, Singapore) was supplemented with 10 % FBS/1% pen/strept, and supplemented with variable amounts of magnesium chloride (Sigma Aldrich, Singapore) to achieve the following concentrations: 0.8 mM, and 8 mM. This shall henceforth be denoted as "proliferative media". "Osteogenic media" was prepared by supplementing various proliferative media with 10 mM β-glycerophosphate, 10⁻⁸ dexamethasone, and 0.2 mM ascorbic acid.

### Cell proliferation and viability

hfMSCs were seeded at a density of 7.5 k/cm² in 6-well plates in the various proliferative and osteogenic medium. At days 3 and 7, AlamarBlue® reagent (Invitrogen, Singapore) was added according to the manufacturer's instruction, and incubated in the dark for 1.5 hours before fluorescence reading at 590 nm with a microplate reader (Spectramax). In addition, cells were stained with fluorescein diacetate (FDA) and propidium iodide (PI) to visualize live and dead cells.

### Alizarin red and von Kossa

hfMSCs were seeded at confluence (20 k/cm²), and cultured in both proliferative and osteogenic media for 14 days. Alizarin red stains were prepared according to the manufacturer's instruction, and maintained at pH 4.2. hfMSCs were fixed with 4 % paraformaldehyde for 5 mins, washed and stained with Alizarin red for 10 mins under gentle shaking. Subsequently, they were thoroughly washed and air-dried before visualization with a microscope, von Kossa staining was done as mentioned earlier.

### ALP and calcium

hfMSCs cultured in both proliferative and osteogenic medium were rinsed with phosphate buffer saline (PBS) solution and incubated in a mixture of collagenase and trypsin for 4 hours at 37 °C. Subsequently, they underwent three freeze-thaw cycles to lyse the cells, and the lysates were evaluated for ALP activity according to the manufacturer's instructions. The pellet obtained was stored separately for evaluating calcium deposition. The pellet obtained previously was dissolved overnight in 0.5 N acetic acid. A calcium assay was then used to quantify the amount of Ca deposited by measuring its absorbance at 612 nm, in accordance with the manufacturer's instructions.

### Real-time polymerase chain reaction

Real-time polymerase chain reaction (RT-PCR) was performed to study the expression of early osteogenic genes by hfMSCs cultured in 6-well plates under proliferative and osteogenic conditions. Total ribonucleic acid (RNA) was harvested by using a Reverse Transcription System (Promega, USA) on days 4 and 8. Next, 1 mg total RNA was reverse-transcribed to complementary deoxyribonucleic acid (cDNA). Finally, the CFX Connect system (BioRad, Singapore) was used to conduct quantitative real-time PCR with TaqMan Universal PCR Master Mix and gene-specific PCR primers including osteocalcin, coll-1, transforming growth factor-beta (TGF-β) and glyceraldehyde-3-phosphate dehydrogenase (GAPDH). Gene expression was normalized to GAPDH by using the comparative 2^{-ΔΔCt} method. The primers used in this experiment are shown in Table 1. All PCRs were carried out in triplicate.

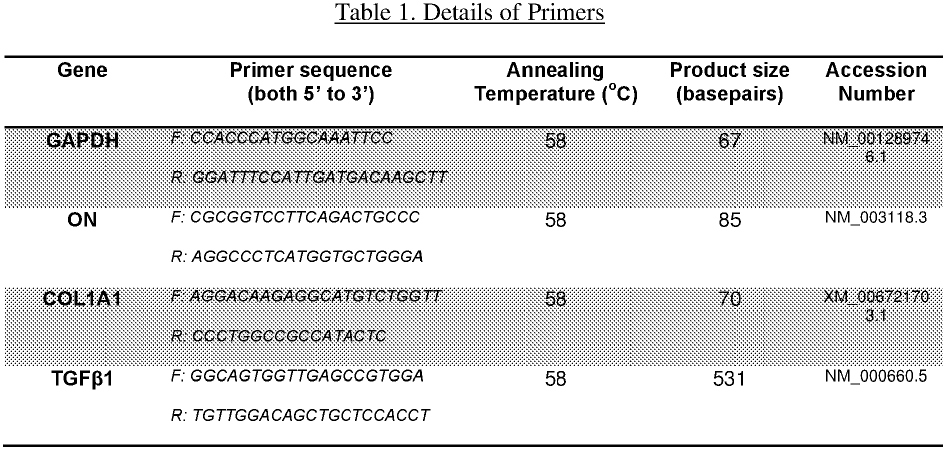

### Fabrication of Mg-releasing PCL films

PCL was placed together with MgCl₂ in a cryomill (Retsch®, Germany). Using similar settings as described elsewhere (Lim J, Chong MS, Chan JK, Teoh SH. Polymer Powder Processing of Cryomilled Polycaprolactone for Solvent-free Generation of Homogeneous Bioactive Tissue Engineering Scaffolds. Small. 2014;17:201302389*),* fine powders of PCL/Mg were generated, and subsequently pressed between two stainless steel sheets to obtain PCL/Mg films with a thickness of 30 to 40 µm. In this example, composites were fabricated using cryomilling. Composites were pre-weighed using a microbalance and loaded into the cryogenic vial with a ball-to-mass ratio of 30:1. The cryomilling protocol was: 6 to 8 min of pre-cooling in liquid nitrogen and 20 min of continuous milling for one cycle. The PCL composite films may be thermally pressed into films of thickness approximately 30 to 60 µm. Briefly, a known mass of composite is placed between two stainless steel sheets on a heat press system with temperature control. Temperature is elevated to 100 °C and pressure added for 30 min. The pressed film is then allowed to cool to room temperature via normal convection cooling. 4 compositions were fabricated: (PCL/Mg) 100/0, 95/5, 90/10, 80/20 The compositions 90/10 and 80/20 are according to the invention.

### Statistics

Student's t-test (two-tailed) was conducted on all data to determine statistical significance. A confidence level of 95 % was taken to be statistically significant, as represented by p < 0.05.

### RESULTS

### Multilineage differentiation potential of hfMSCs

hfMSCs were shown here to be able to differentiate into the following three lineages: adipogenic, chondrogenic, and osteogenic (FIG. 9), a result in agreement with previous reports (Zhang ZY, Teoh SH, Chong MSK, Schantz JT, Fisk NM, Choolani MA, et al. Superior Osteogenic Capacity for Bone Tissue Engineering of Fetal Compared with Perinatal and Adult Mesenchymal Stem Cells. Stem Cells. 2009;27:126-37*).* Accordingly, hfMSCs that were differentiated down the adipogenic pathway presented lipid vacuoles; chondrogenic hfMSCs displayed positive staining for Safranin O; osteogenic hfMSCs displayed positive staining for von Kossa.

### Validating the source of Mg

The sources of Mg were evaluated to determine their suitability for this study. From the results, both MgCl₂ and MgSO₄ allowed proliferation and differentiation of hfMSCs (FIG. 10).

### Effect of various Mg levels on hfMSC proliferation

hfMSC proliferation in the Mg free and 8 mM groups were compared and normalized against the basal level (0.8 mM), in both proliferative and osteogenic media. In both proliferative and osteogenic media, Mg starvation suppressed cell growth to a particularly large extent (FIG. 11; p < 0.001). On the other hand, 8 mM of Mg supported cell proliferation (p < 0.001). Corresponding visualization with live/dead (FDA/PI) imaging led to corroborating results, with higher Mg indicating higher hfMSC proliferation (FIG. 11).

### Effect of Mg on osteogenic differentiation

hfMSCs cultured under prolonged exposure to high levels of Mg (8 mM) exhibited lower levels of ON, coll-1, and TGF-β expressions (FIG. 12). Upon switching to Mg-free conditions after 4 days, hfMSCs demonstrated higher potential for osteogenic differentiation as compared to 0.8 mM.

### Osteocalcin protein expression

Expression of osteocalcin (OC) protein was determined using immunocytochemical staining. From the results (FIG. 13), OC expression was clearly demonstrated in the group exposed to decreasing concentrations of Mg, while prolonged exposure to Mg resulted in suppressed expression of OC from the hfMSCs.

### Discussion

The intriguing role of Mg in directing osteogenesis has been of recent interest, due to the seemingly phenomenological observations of enhanced osseointegration in coated implants. Given that Mg is complexed to adenosine triphosphate (ATP), which is ternary complex of the catalytic subunit of cAMP-dependent protein kinase, it is understandable that Mg plays an important role in regulating many cellular processes, including that of cell adhesion to substrates. However, its purported role in osteogenesis remains profound knowledge, and an attempt was made in this study to understand its importance by hypothesizing the temporal effect of Mg on osteogensis.

First, it is attempted to understand the effect of various sources of Mg on MSC proliferation. It was established and demonstrated that soluble magnesium salts including MgCl₂ and MgSO₄ were suitable. On this note, the use of MgCl₂ and MgSO₄ supplementation for the study of osteogenesis has previously been validated. Thereafter, it was shown here that higher levels of Mg (8 mM) resulted in higher MSC proliferation over time in both culture and osteogenic medium while the lack of Mg (Mg-free) resulted in inhibited cell proliferation. This was similarly reported in human osteoblast-like cells (MG-63, SaOS, and U2-OS), clearly cementing the role of Mg in DNA and protein synthesis through melastatin-like transient receptor potential 6 and 7 (TRPM6 and 7). More accurately, studies have shown that the mammalian target of rapamycin (mTOR), a protein kinase in the PI3-K pathway, is regulated by MgATP. These studies, together with the present results, verified and confirmed that Mg has a positive influence on MSC proliferation.

In the presence of soluble osteogenic factors such as dexamethasone and β-glycerophosphate, MSCs already have a strong predisposition towards the osteogenic lineage. When further supplemented with higher Mg (8 mM), characteristic hallmarks of osteogenesis such as ALP activity and calcium deposition were further upregulated. Over 7 days, it was demonstrated temporal expression of ALP in response to varying Mg concentrations, with ALP expression peaking on day 3 in 8 mM of Mg as compared to basal levels (0.8 mM), which possibly occurred either on day 7 or beyond. In tandem with ALP expression on day 3 in the 8 mM Mg group, calcium deposition was significantly expressed on day 7. From the literature, Leem et al. (Leem Y-H, Lee K-S, Kim J-H, Seok H-K, Chang J-S, Lee D-H. Magnesium ions facilitate integrin alpha 2- and alpha 3-mediated proliferation and enhance alkaline phosphatase expression and activity in hBMSCs. Journal of Tissue Engineering and Regenerative Medicine. 2014; doi:10.1002/term.1861*)* also reported enhanced ALP activity within the first 72 hours (3 days) in the presence of 2.5 mM of Mg. While the expression of ALP is understandably transient, it is an important, early indication of osteogenesis. ALP may traditionally be known as pyrophosphatase, which is an enzyme that is responsible for the production of inorganic phosphate, which is then transported through the cell membrane via vesicles for interaction with available, unbound calcium ions to form calcium phosphate (CaP) crystals.

According to a report by Li et al. *(*Li RW, Kirkland NT, Truong J, Wang J, Smith PN, Birbilis N, et al. The influence of biodegradable magnesium alloys on the osteogenic differentiation of human mesenchymal stem cells. Journal of Biomedical Materials Research Part A. 2014), hfMSCs proliferated well in the presence of 0.5-0.8 mM of Mg, while at high levels of Mg (ca. 5-8 mM), they showed poorer proliferation. On the other hand, when hfMSCs were exposed to higher levels of Mg, their differentiation towards the osteogenic phenotype was increased (14 day culture), which is in agreement with the present results. However, the medium extracts used in the previous study were diluted with other alloying metals, possibly resulting in the delayed onset of ALP activity. In the present study, supplementation of Mg to culture medium was a more direct way of understanding its effects on MSC differentiation, to which it was demonstrated an earlier peak in ALP expression on day 3.

To understand the effect of decreasing local Mg concentration over time (as per *in vivo* orthopaedic implants), hfMSCs were cultured in 8 mM of Mg over 4 days, before switching to 0.8 mM and Mg-free conditions. Present results demonstrated that the osteogenic potential of MSCs was significantly upregulated with decreasing concentrations of Mg due to the upregulation of osteogenic genes such as ON, coll-I, and TGF-β. On the other hand, prolonged exposure of hfMSCs to elevated Mg did not result in increased osteogenic activity, which is in agreement with a previous study by Leidi et al. (Leidi M, Dellera F, Mariotti M, Maier JA. High magnesium inhibits human osteoblast differentiation in vitro. Magnes Res. 2011;24:1-6*)* and Yang et al. (Yang C, Yuan G, Zhang J, Tang Z, Zhang X, Dai K. Effects of magnesium alloys extracts on adult human bone marrow-derived stromal cell viability and osteogenic differentiation. Biomedical Materials. 2010;5:045005). In the latter study by Yang et al., human bone marrow MSCs (bMSCs) maintained in culture extracts taken from Mg alloys (AZ91D, NZ30K) and Mg metals demonstrated upregulation of osteopontin (OPN) at day 6 at the transcription level but not at the protein level. ALP levels were also similar to control (no added Mg) throughout the study period. These evidences, taken in consideration with the present observation here, suggest that transient exposure to elevated levels of Mg may potentiate early differentiation of hfMSCs.

### Conclusion

In summary of this example, the response of hfMSCs to different levels of Mg was studied, with the aim of understanding the positive effect of Mg-coated orthopaedic implants. It is hypothesized and demonstrated that transient exposure to elevated levels of Mg led to significant upregulation of osteogenic genes and proteins, leading to substantial calcium deposition. These results are likely to facilitate understanding of the observations related to the osteogenic effects of Mg-coated implants *in vivo.*

By "comprising" it is meant including, but not limited to, whatever follows the word "comprising". Thus, use of the term "comprising" indicates that the listed elements are required or mandatory, but that other elements are optional and may or may not be present.

By "consisting of' is meant including, and limited to, whatever follows the phrase "consisting of'. Thus, the phrase "consisting of' indicates that the listed elements are required or mandatory, and that no other elements may be present.

By "about" in relation to a given numerical value, such as for temperature and period of time, it is meant to include numerical values within 10% of the specified value.

Other embodiments are within the following claims and non- limiting examples. In addition, where features or aspects of the disclosure are described in terms of Markush groups, those skilled in the art will recognize that the disclosure is also thereby described in terms of any individual member or subgroup of members of the Markush group.

### REFERENCES

1. Cao, L., Wang, J., Hou, J., Xing, W. & Liu, C. Vascularization and bone regeneration in a critical sized defect using 2-N,6-O-sulfated chitosan nanoparticles incorporating BMP-2. Biomaterials 35, 684-698 (2014).
2. Liu, Y.C. et al. Contrasting Effects of Vasculogenic Induction Upon Biaxial Bioreactor Stimulation of Mesenchymal Stem Cells and Endothelial Progenitor Cells Cocultures in Three-Dimensional Scaffolds Under In Vitro and In Vivo Paradigms for Vascularized Bone Tissue Engineering. Tissue Engineering Part A 19, 893-904 (2013).
3. Liu, Y., Chan, J.K.Y. & Teoh, S.H. Review of vascularised bone tissue-engineering strategies with a focus on co-culture systems. Journal of Tissue Engineering and Regenerative Medicine (2012).
4. Hollister, S.J. Porous scaffold design for tissue engineering. Nature Materials 4, 518-524 (2005).
5. Fawcett, W.J., Haxby, E.J. & Male, D.A. Magnesium: physiology and pharmacology. Br. J. Anaesth. 83, 302-320 (1999).
6. RK, R. Magnesium deficiency: A cause of heterogeneous disease in humans. J. Bone Miner. Res. 13, 749-758 (1998).
7. McLean, R.M. Magnesium and its therapeutic uses: a review. The American journal of medicine 96, 63-76 (1994).
8. Boskey, A.L. et al. Effect of short-term hypomagnesemia on the chemical and mechanical properties of rat bone. J. Orthop. Res. 10, 774-783 (1992).
9. Agus, Z. & Morad, M. Modulation of cardiac ion channels by magnesium. Annu. Rev. Physiol. 53, 299-307 (1991).
10. Vernon, W. The role of magnesium in nucleic-acid and protein metabolism. Magnesium 7, 234 (1988).
11. Elin, R.J. Magnesium metabolism in health and disease. Dis. Mon. 34, 166-218 (1988).
12. PO, W. Magnesium. Am. J. Clin. Nutr. 45, 1305-1312 (1987).
13. Rubin, H. Central role for magnesium in coordinate control of metabolism and growth in animal cells. Proceedings of the National Academy of Sciences 72, 3551-3555 (1975).
14. Zofkova, I., Nemcikova, P. & Matucha, P. Trace elements and bone health. Clin Chem Lab Med. 51, 1555-1561. doi: 1510.1515/cclm-2012-0868. (2013).
15. Kirkland, N.T. Magnesium biomaterials: past, present and future. Corrosion Engineering, Science and Technology 47, 322-328 (2012).
16. Rude, R.K., Singer, F.R. & Gruber, H.E. Skeletal and Hormonal Effects of Magnesium Deficiency. J. Am. Coll. Nutr. 28, 131-141 (2009).
17. Park, J.-W., Ko, H.-J., Jang, J.-H., Kang, H. & Suh, J.-Y. Increased new bone formation with a surface magnesium-incorporated deproteinized porcine bone substitute in rabbit calvarial defects. Journal of Biomedical Materials Research Part A 100A, 834-840 (2012).
18. Ozturk, C.F., Karakelleoglu, C., Orbak, Z. & Yildiz, L. The Effect of Serum Magnesium Levels and Serum Endothelin-1 Levels on Bone Mineral Density in Protein Energy Malnutrition. West Indian Med. J. 61, 213-218 (2012).
19. Lakhkar, N.J. et al. Bone formation controlled by biologically relevant inorganic ions: Role and controlled delivery from phosphate-based glasses. Advanced Drug Delivery Reviews (2012).
20. Khandaker, M., Duggan, K. & Perram, M. in, Vol. 17. (ed. T. Proulx) 295-299 (Springer New York, 2011).
21. Kraus, T. et al. Magnesium alloys for temporary implants in osteosynthesis: in vivo studies of their degradation and interaction with bone. Acta Biomaterialia 8, 1230-1238 (2012).
22. Song, G. Control of biodegradation of biocompatable magnesium alloys. Corrosion Science 49, 1696-1701 (2007).
23. Witte, F. et al. In vivo corrosion of four magnesium alloys and the associated bone response. Biomaterials 26, 3557-3563 (2005).
24. Xu, L., Yu, G., Zhang, E., Pan, F. & Yang, K. In vivo corrosion behavior of Mg-Mn-Zn alloy for bone implant application. Journal of Biomedical Materials Research Part A 83, 703-711 (2007).
25. Yazdimamaghani, M., Razavi, M., Vashaee, D. & Tayebi, L. Development and degradation behavior of magnesium scaffolds coated with polycaprolactone for bone tissue engineering. Materials Letters 132, 106-110 (2014).
26. Muguruma, Y. et al. Reconstitution of the functional human hematopoietic microenvironment derived from human mesenchymal stem cells in the murine bone marrow compartment. Blood 107, 1878-1887 (2006).
27. Méndez-Ferrer, S. et al. Mesenchymal and haematopoietic stem cells form a unique bone marrow niche. Nature 466, 829-834 (2010).
28. Bigg, D. Rheological analysis of highly loaded polymeric composites filled with non-agglomerating spherical filler particles. Polymer Engineering & Science 22, 512-518 (1982).
29. Hong, J.-H. et al. TAZ, a transcriptional modulator of mesenchymal stem cell differentiation. Science 309, 1074-1078 (2005).
30. Olivares-Navarrete, R. et al. Direct and indirect effects of microstructured titanium substrates on the induction of mesenchymal stem cell differentiation towards the osteoblast lineage. Biomaterials 31, 2728-2735 (2010).
31. Foster, L.J. et al. Differential expression profiling of membrane proteins by quantitative proteomics in a human mesenchymal stem cell line undergoing osteoblast differentiation. Stem Cells 23, 1367-1377 (2005).
32. Biotech, K., Vol. 20142011).
33. Garcia-Godoy, F. & Murray, P.E. Status and potential commercial impact of stem cell-based treatments on dental and craniofacial regeneration. Stem cells and development 15, 881-887 (2006).
34. Nieves JW. Bone: Maximizing bone health [mdash] magnesium, BMD and fractures. Nature Reviews Endocrinology. 2014;10:255-6.
35. Castiglioni S, Cazzaniga A, Albisetti W, Maier JA. Magnesium and osteoporosis: current state of knowledge and future research directions. Nutrients. 2013;5:3022-33.
36. Quinn SJ, Thomsen AR, Egbuna O, Pang J, Baxi K, Goltzman D, et al. CaSR-mediated interactions between calcium and magnesium homeostasis in mice. American Journal of Physiology-Endocrinology and Metabolism. 2013;304:E724-E33.
37. Rubin H. Central role for magnesium in coordinate control of metabolism and growth in animal cells. Proceedings of the National Academy of Sciences. 1975;72:3551-5.
38. Solomons C, Styner J. Osteogenesis imperfecta: effect of magnesium administration on pyrophosphate metabolism. Calcif Tissue Res. 1969;3:318-26.
39. Gorgels TG, Waarsing JH, de Wolf A, Jacoline B, Loves WJ, Bergen AA. Dietary magnesium, not calcium, prevents vascular calcification in a mouse model for pseudoxanthoma elasticum. J Mol Med. 2010;88:467-75.
40. Salem S, Bruck H, Bahlmann FH, Peter M, Passlick-Deetjen J, Kretschmer A, et al. Relationship between magnesium and clinical biomarkers on inhibition of vascular calcification. Am J Nephrol. 2011;35:31-9.
41. Staiger MP, Pietak AM, Huadmai J, Dias G. Magnesium and its alloys as orthopedic biomaterials: A review. Biomaterials. 2006;27:1728-34.
42. Zreiqat H, Howlett CR, Zannettino A, Evans P, Schulze-Tanzil G, Knabe C, et al. Mechanisms of magnesium-stimulated adhesion of osteoblastic cells to commonly used orthopaedic implants. J Biomed Mater Res. 2002;62:175-84.
43. Yamasaki Y, Yoshida Y, Okazaki M, Shimazu A, Kubo T, Akagawa Y, et al. Action of FGMgC03Ap-collagen composite in promoting bone formation. Biomaterials. 2003;24:4913-20.
44. Witte F, Feyerabend F, Maier P, Fischer J, Störmer M, Blawert C, et al. Biodegradable magnesium-hydroxyapatite metal matrix composites. Biomaterials. 2007;28:2163-74.
45. Wong HM, Yeung KW, Lam KO, Tam V, Chu PK, Luk KD, et al. A biodegradable polymer-based coating to control the performance of magnesium alloy orthopaedic implants. Biomaterials. 2010;31:2084-96.
46. Shadanbaz S, Walker J, Staiger MP, Dias GJ, Pietak A. Growth of calcium phosphates on magnesium substrates for corrosion control in biomedical applications via immersion techniques. Journal of Biomedical Materials Research Part B: Applied Biomaterials. 2013;101:162-72.
47. Park M, Lee JE, Park CG, Lee SH, Seok HK, Choy YB. Polycaprolactone coating with varying thicknesses for controlled corrosion of magnesium. Journal of Coatings Technology and Research. 2013;10:695-706.
48. Jo J-H, Li Y, Kim S-M, Kim H-E, Koh Y-H. Hydroxyapatite/poly (ε-caprolactone) double coating on magnesium for enhanced corrosion resistance and coating flexibility. J Biomater Appl. 2013;28:617-25.
49. Yoshizawa S, Brown A, Barchowsky A, Sfeir C. Magnesium ion stimulation of bone marrow stromal cells enhances osteogenic activity, simulating the effect of magnesium alloy degradation. Acta Biomaterialia. 2014.
50. Li RW, Kirkland NT, Truong J, Wang J, Smith PN, Birbilis N, et al. The influence of biodegradable magnesium alloys on the osteogenic differentiation of human mesenchymal stem cells. Journal of Biomedical Materials Research Part A. 2014.
51. Boskey A, Posner A. Magnesium stabilization of amorphous calcium phosphate: akinetic study. Materials Research Bulletin. 1974;9:907-16.
52. Blumenthal N, Betts F, Posner A. Stabilization of amorphous calcium phosphate by Mg and ATP. Calcif Tissue Res. 1977;23:245-50.
53. Lakhkar NJ, Lee I-H, Kim H-W, Salih V, Wall IB, Knowles JC. Bone formation controlled by biologically relevant inorganic ions: Role and controlled delivery from phosphate-based glasses. Advanced Drug Delivery Reviews. 2012.
54. Zhang Z-Y, Teoh S-H, Chong MSK, Lee ESM, Tan L-G, Mattar CN, et al. Neo-vascularization and bone formation mediated by fetal mesenchymal stem cell tissue-engineered bone grafts in critical-size femoral defects. Biomaterials. 2010;31:608-20.
55. Lim J, Chong MS, Chan JK, Teoh SH. Polymer Powder Processing of Cryomilled Polycaprolactone for Solvent-free Generation of Homogeneous Bioactive Tissue Engineering Scaffolds. Small. 2014;17:201302389.
56. Zhang ZY, Teoh SH, Chong MSK, Schantz JT, Fisk NM, Choolani MA, et al. Superior Osteogenic Capacity for Bone Tissue Engineering of Fetal Compared with Perinatal and Adult Mesenchymal Stem Cells. Stem Cells. 2009;27:126-37.
57. Zheng J, Knighton DR, Ten Eyck LF, Karlsson R, Xuong N-H, Taylor SS, et al. Crystal structure of the catalytic subunit of cAMP-dependent protein kinase complexed with magnesium-ATP and peptide inhibitor. Biochemistry (Mosc). 1993;32:2154-61.
58. Takeichi M, Okada TS. Roles of magnesium and calcium ions in cell-to-substrate adhesion. Exp Cell Res. 1972;74:51-60.
59. Wu L, Luthringer BJ, Feyerabend F, Schilling AF, Willumeit R. Effects of extracellular magnesium on the differentiation and function of human osteoclasts. Acta biomaterialia. 2014;10:2843-54.
60. Abed E, Moreau R. Importance of melastatin - like transient receptor potential 7 and cations (magnesium, calcium) in human osteoblast - like cell proliferation. Cell Prolif. 2007;40:849-65.
61. Schlingmann KP, Waldegger S, Konrad M, Chubanov V, Gudermann T. TRPM6 and TRPM7--Gatekeepers of human magnesium metabolism. Biochim Biophys Acta. 2007;1772:813-21.
62. Rubin H. Magnesium: The missing element in molecular views of cell proliferation control. Bioessays. 2005;27:311-20.
63. Leem Y-H, Lee K-S, Kim J-H, Seok H-K, Chang J-S, Lee D-H. Magnesium ions facilitate integrin alpha 2- and alpha 3-mediated proliferation and enhance alkaline phosphatase expression and activity in hBMSCs. Journal of Tissue Engineering and Regenerative Medicine. 2014; doi:10.1002/term.1861
64. Siffert RS. The role of alkaline phosphatase in osteogenesis. The Journal of experimental medicine. 1951;93:415-26.
65. Leidi M, Dellera F, Mariotti M, Maier JA. High magnesium inhibits human osteoblast differentiation in vitro. Magnes Res. 2011;24:1-6.
66. Yang C, Yuan G, Zhang J, Tang Z, Zhang X, Dai K. Effects of magnesium alloys extracts on adult human bone marrow-derived stromal cell viability and osteogenic differentiation. Biomedical Materials. 2010;5:045005.

## Claims

1. A method for forming a biocomposite thin film or three-dimensional (3D) scaffold comprising a polymeric matrix and a magnesium filler incorporated into the polymeric matrix, wherein the magnesium filler comprises 10 wt%, 11 wt%, 12% wt%, 13% wt%, 14% wt%, 15% wt%, 16% wt%, 17% wt%, 18% wt%, 19% wt%, or 20 wt% based on the total weight of the biocomposite, of a soluble magnesium salt that leaches or dissolves upon contact with body fluid to create a biocomposite thin film or three-dimensional (3D) scaffold with gradually increasing porosity, the method comprising:
mixing the polymeric matrix and magnesium filler;
processing the mixture of the polymeric matrix and magnesium filler in a cryomill to obtain fine powder without a solvent or heat;
processing the fine powder to form the biocomposite thin film or the biocomposite three-dimensional (3D) scaffold; and
wherein processing the mixture of the polymeric matrix and magnesium filler in a cryomill to obtain fine powder comprises loading pre-weighed mixture into a cryogenic vial with a ball-to-mass ratio of 30:1, pre-cooling the cryogenic vial in liquid nitrogen for 6 to 8 minutes, and continuous milling for one cycle for 20 minutes.

2. The method of claim 1, wherein the biocomposite thin film is formed by thermally pressing the fine powder between two stainless steel sheets in a heat press system
and preferably the fine powder are thermally pressed at 100 °C with pressure applied for a period of time, followed by cooling the pressed film to room temperature.

3. The method of claim 1, wherein the 3D biocomposite scaffold is formed by an additive manufacturing technique, or using a die set along with the incorporation of 50 vol% of sodium chloride, followed by leaching in water.

4. The method of any one of claims 1 to 3, wherein the magnesium filler does not comprise a magnesium alloy or elemental magnesium.

5. The method of any one of claims 1 to 4, wherein the magnesium salt comprises magnesium chloride (MgCl₂), magnesium sulphate (MgSO₄), or magnesium phosphate (Mg₃(PO₄)₂).

6. The method of any one of claims 1 to 5, wherein the polymeric matrix comprises a polymer selected from the group consisting of polycaprolactone (PCL), poly(lactic-co-glycolic acid) (PLGA), poly(lactic acid) (PLA), poly(glycolic acid) (PGA), the family of polyhydroxyalkanoates (PHA), polyethylene glycol (PEG), polypropylene glycol (PPG), polyesteramide (PEA), poly(lactic acid-co-caprolactone), poly(lactide-co-trimethylene carbonate), poly(sebacic acid-co-ricinoleic acid) and a combination thereof.

7. A biocomposite thin film or three-dimensional (3D) scaffold produced by the method of any one of claims 1 to 6, for use in promoting bone growth and repair, regeneration, and/or proliferation of host tissues, wherein the biocomposite thin film or three-dimensional (3D) scaffold is to be implanted at a site in need of bone growth and repair, regeneration, and/or proliferation of host tissues, wherein the biocomposite thin film or three-dimensional (3D) scaffold comprises a polymeric matrix comprising polycaprolactone (PCL), and a magnesium filler incorporated into the polymeric matrix, and wherein the magnesium filler comprises 10 wt%, 11 wt%, 12% wt%, 13% wt%, 14% wt%, 15% wt%, 16% wt%, 17% wt%, 18% wt%, 19% wt%, or 20 wt% based on the total weight of the biocomposite, of a magnesium salt that leaches or dissolves upon contact with body fluid such that the biocomposite thin film or three-dimensional (3D) scaffold has gradually increasing porosity.

8. The biocomposite thin film or three-dimensional (3D) scaffold for use of claim 7, wherein the magnesium filler does not comprise a magnesium alloy or elemental magnesium.

9. The biocomposite thin film or three-dimensional (3D) scaffold for use of claim 7 or 8, wherein the magnesium salt comprises magnesium chloride (MgCl₂), magnesium sulphate (MgSO₄), or magnesium phosphate (Mg₃(PO₄)₂).

10. A biocomposite thin film or three-dimensional (3D) scaffold produced by the method of any one of claims 1 to 6, comprising a polymeric matrix comprising polycaprolactone (PCL), and a magnesium filler incorporated into the polymeric matrix, wherein the magnesium filler comprises 10 wt%, 11 wt%, 12% wt%, 13% wt%, 14% wt%, 15% wt%, 16% wt%, 17% wt%, 18% wt%, 19% wt%, or 20 wt% based on the total weight of the biocomposite, of a magnesium salt that leaches or dissolves upon contact with body fluid to create a biocomposite thin film or three-dimensional (3D) scaffold with gradually increasing porosity.

11. The biocomposite thin film or three-dimensional (3D) scaffold of claim 10, wherein the magnesium filler does not comprise a magnesium alloy or elemental magnesium.

12. The biocomposite thin film or three-dimensional (3D) scaffold of claim 10 or 11, wherein the magnesium salt comprises magnesium chloride (MgCl₂), magnesium sulphate (MgSO₄), or magnesium phosphate (Mg₃(PO₄)₂).

## Patentansprüche

1. Verfahren zum Ausbilden eines Bioverbundwerkstoff-Dünnfilms oder eines dreidimensionalen (3D-) Bioverbundwerkstoff-Gerüsts, der/das eine Polymermatrix und einen in die Polymermatrix integrierten Magnesium-Füllstoff umfasst, wobei der Magnesium-Füllstoff 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-% oder 20 Gew.-%, bezogen auf das Gesamtgewicht des Bioverbundwerkstoffs, eines löslichen Magnesiumsalzes umfasst, das bei Kontakt mit Körperflüssigkeit auslaugt oder gelöst wird, wobei ein Bioverbundwerkstoff-Dünnfilm oder ein dreidimensionales (3D-) Bioverbundwerkstoff-Gerüst mit graduell ansteigender Porosität gebildet wird, wobei das Verfahren Folgendes umfasst:
das Vermischen der Polymermatrix und des Magnesium-Füllstoffs;
das Verarbeiten des Gemischs aus Polymermatrix und Magnesium-Füllstoff in einer kryogenen Mühle, um ohne Lösungsmittel oder Hitze ein feines Pulver zu erhalten;
das Verarbeiten des feinen Pulvers zur Ausbildung des Bioverbundwerkstoff-Dünnfilms oder des dreidimensionalen (3D-) Bioverbundwerkstoff-Gerüsts; und
wobei das Verarbeiten des Gemischs aus Polymermatrix und Magnesium-Füllstoff in einer kryogenen Mühle zum Erhalt des feinen Pulvers das Einfüllen eines vorab eingewogenen Gemischs in eine kryogene Phiole mit einem Kugel-zu-Masse-Verhältnis von 30:1, das 6- bis 8-minütige Vorkühlen der kryogenen Phiole in flüssigem Stickstoff und das kontinuierliche Mahlen in einem 20-minütigen Zyklus umfasst.

2. Verfahren nach Anspruch 1, wobei der Bioverbundwerkstoff-Dünnfilm durch Heißpressen des feinen Pulvers zwischen zwei Edelstahlplatten in einem Heißpresssystem ausgebildet wird,
wobei das feine Pulver vorzugsweise bei 100 °C heißgepresst wird, wobei für einen Zeitraum Druck angelegt wird, worauf das Abkühlen des gepressten Films auf Raumtemperatur folgt.

3. Verfahren nach Anspruch 1, wobei das 3D-Bioverbundwerkstoff-Gerüst durch ein additives Fertigungsverfahren oder unter Verwendung eines Düsensatzes in Verbindung mit dem Miteinbeziehen von 50 Vol.-% Natriumchlorid, gefolgt vom Auslaugen in Wasser ausgebildet wird.

4. Verfahren nach einem der Ansprüche 1 bis 3, wobei der Magnesium-Füllstoff keine Magnesiumlegierung und kein elementares Magnesium umfasst.

5. Verfahren nach einem der Ansprüche 1 bis 4, wobei das Magnesiumsalz Magnesiumchlorid (MgCl₃), Magnesiumsulfat (M_{g}SO₄) oder Magnesiumphosphat (Mg₃(PO₄)₂) umfasst.

6. Verfahren nach einem der Ansprüche 1 bis 5, wobei die Polymermatrix ein Polymer, ausgewählt aus der aus Polycaprolacton (PCL), Poly(milchsäure-co-glykolsäure) (PLGA), Poly(milchsäure) (PLA), Poly(glykolsäure) (PGA), der Familie der Polyhydroxyalkanoate (PHA), Polyethylenglykol (PEG), Polypropylenglykol (PPG), Polyesteramid (PEA), Poly-(milchsäure-co-caprolacton), Poly(lactid-co-trimethylencarbonat), Poly(sebacinsäure-co-ricinolsäure) und Kombinationen davon bestehenden Gruppe ist.

7. Bioverbundwerkstoff-Dünnfilm oder dreidimensionales (3D-) Bioverbundwerkstoff-Gerüst, hergestellt durch ein Verfahren nach einem der Ansprüche 1 bis 6, zur Verwendung zur Förderung von Knochenwachstum und -reparatur, Regeneration und/oder Proliferation von Wirtsgewebe, wobei der Bioverbundwerkstoff-Dünnfilm oder das dreidimensionale (3D-) Bioverbundwerkstoff-Gerüst an einer Stelle zu implantieren ist, an der Knochenwachstum und -reparatur, Regeneration und/oder Proliferation von Wirtsgewebe erforderlich ist/sind, wobei der Bioverbundwerkstoff-Dünnfilm oder das dreidimensionale (3D-) Bioverbundwerkstoff-Gerüst eine Polymermatrix, die Polycaprolacton (PCL) umfasst, und einen in die Polymermatrix integrierten Magnesium-Füllstoff umfasst, und wobei der Magnesium-Füllstoff 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-% oder 20 Gew.-%, bezogen auf das Gesamtgewicht des Bioverbundwerkstoffs, eines löslichen Magnesiumsalzes umfasst, das bei Kontakt mit Körperflüssigkeit auslaugt oder gelöst wird, wobei ein Bioverbundwerkstoff-Dünnfilm oder ein dreidimensionales (3D-) Bioverbundwerkstoff-Gerüst mit graduell ansteigender Porosität gebildet wird.

8. Bioverbundwerkstoff-Dünnfilm oder dreidimensionales (3D-) Bioverbundwerkstoff-Gerüst zur Verwendung nach Anspruch 7, wobei der Magnesium-Füllstoff keine Magnesiumlegierung und kein elementares Magnesium umfasst.

9. Bioverbundwerkstoff-Dünnfilm oder dreidimensionales (3D-) Bioverbundwerkstoff-Gerüst zur Verwendung nach Anspruch 7 oder 8, wobei das Magnesiumsalz Magnesiumchlorid (MgCl₂), Magnesiumsulfat (M_{g}SO₄) oder Magnesiumphosphat (Mg₃(PO₄)₂) umfasst.

10. Bioverbundwerkstoff-Dünnfilm oder dreidimensionales (3D-) Bioverbundwerkstoff-Gerüst, hergestellt durch ein Verfahren nach einem der Ansprüche 1 bis 6, der/das eine Polymermatrix, die Polycaprolacton (PCL) umfasst, und einen in die Polymermatrix integrierten Magnesium-Füllstoff umfasst, wobei der Magnesium-Füllstoff 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-% oder 20 Gew.-%, bezogen auf das Gesamtgewicht des Bioverbundwerkstoffs, eines löslichen Magnesiumsalzes umfasst, das bei Kontakt mit Körperflüssigkeit auslaugt oder gelöst wird, wobei ein Bioverbundwerkstoff-Dünnfilm oder ein dreidimensionales (3D-) Bioverbundwerkstoff-Gerüst mit graduell ansteigender Porosität gebildet wird.

11. Bioverbundwerkstoff-Dünnfilm oder dreidimensionales (3D-) Bioverbundwerkstoff-Gerüst nach Anspruch 10, wobei der Magnesium-Füllstoff keine Magnesiumlegierung und kein elementares Magnesium umfasst.

12. Bioverbundwerkstoff-Dünnfilm oder dreidimensionales (3D-) Bioverbundwerkstoff-Gerüst nach Anspruch 10 oder 11, wobei das Magnesiumsalz Magnesiumchlorid (MgCl₂), Magnesiumsulfat (M_{g}SO₄) oder Magnesiumphosphat (Mg₃(PO₄)₂) umfasst.

## Revendications

1. Procédé pour former un film mince biocomposite ou un échafaudage tridimensionnel (3D) comprenant une matrice polymère et une charge de magnésium incorporée dans la matrice polymère, dans lequel la charge de magnésium comprend 10 % en poids, 11 % en poids, 12 % en poids, 13 % en poids, 14 % en poids, 15 % en poids, 16 % % en poids, 17 % en poids, 18 % en poids, 19 % en poids ou 20 % en poids, sur la base du poids total du biocomposite, d'un sel de magnésium soluble qui se lixivie ou se dissout au contact d'un fluide corporel pour créer un film mince biocomposite ou un échafaudage tridimensionnel (3D) avec une porosité augmentant graduellement, le procédé comprenant les étapes consistant à :
mélanger la matrice polymère et la charge de magnésium ;
traiter le mélange de la matrice polymère et de la charge de magnésium dans un broyeur cryogénique pour obtenir une poudre fine sans solvant ni chaleur ;
traiter la poudre fine pour former le film mince biocomposite ou l'échafaudage tridimensionnel (3D) biocomposite ; et
dans lequel le traitement du mélange de la matrice polymère et de la charge de magnésium dans un broyeur cryogénique pour obtenir une poudre fine comprend un chargement du mélange pré-pesé dans un flacon cryogénique avec un rapport bille/masse de 30:1, un pré-refroidissement du flacon cryogénique dans de l'azote liquide pendant 6 à 8 minutes, et un broyage continu pendant un cycle de 20 minutes.

2. Procédé selon la revendication 1, dans lequel le film mince biocomposite est formé en pressant thermiquement la poudre fine entre deux feuilles d'acier inoxydable dans un système de presse à chaud
et de préférence la poudre fine est pressée thermiquement à 100°C avec une pression appliquée pendant une période de temps, suivi d'un refroidissement du film pressé à température ambiante.

3. Procédé selon la revendication 1, dans lequel l'échafaudage biocomposite 3D est formé par une technique de fabrication additive, ou en utilisant un ensemble de matrices avec l'incorporation de 50 % en volume de chlorure de sodium, avant une lixiviation dans l'eau.

4. Procédé selon l'une quelconque des revendications 1 à 3, dans lequel la charge de magnésium ne comprend pas d'alliage de magnésium ni de magnésium élémentaire.

5. Procédé selon l'une quelconque des revendications 1 à 4, dans lequel le sel de magnésium comprend du chlorure de magnésium (MgCl₂), du sulfate de magnésium (MgSO₄) ou du phosphate de magnésium (Mg₃(PO₄)₂).

6. Procédé selon l'une quelconque des revendications 1 à 5, dans lequel la matrice polymère comprend un polymère choisi dans le groupe comprenant polycaprolactone (PCL), poly(acide lactique-co-glycolique) (PLGA), poly(acide lactique) (PLA), poly(acide glycolique) (PGA), la famille des polyhydroxyalcanoates (PHA), polyéthylène glycole (PEG), polypropylène glycole (PPG), polyesteramide (PEA), poly(acide lactique-co-caprolactone), poly(carbonate de lactide-co-triméthylène), poly(acide sébacique-acide co-ricinoléique), et une combinaison de ceux-ci.

7. Film mince biocomposite ou échafaudage tridimensionnel (3D) produit par le procédé de l'une quelconque des revendications 1 à 6, destiné à être utilisé pour favoriser une croissance osseuse et une réparation, une régénération et/ou une prolifération de tissus hôtes, dans lequel le film mince biocomposite ou l'échafaudage tridimensionnel (3D) doit être implanté au niveau d'un site nécessitant une croissance osseuse et une réparation, une régénération et/ou une prolifération de tissus hôtes, dans lequel le film mince biocomposite ou l'échafaudage tridimensionnel (3D) comprend une matrice polymère comprenant de la polycaprolactone (PCL), et une charge de magnésium incorporée dans la matrice polymère, et dans lequel la charge de magnésium comprend 10 % en poids, 11 % en poids, 12 % en poids, 13 % en poids, 14 % en poids, 15 % en poids, 16 % en poids, 17 % en poids, 18 % en poids, 19 % en poids ou 20 % en poids, sur la base du poids total du biocomposite, d'un sel de magnésium qui se lixivie ou se dissout au contact d'un fluide corporel de telle sorte que le film mince biocomposite ou l'échafaudage tridimensionnel (3D) a une porosité augmentant graduellement.

8. Film mince biocomposite ou échafaudage tridimensionnel (3D) à utiliser selon la revendication 7, dans lequel la charge de magnésium ne comprend pas d'alliage de magnésium ni de magnésium élémentaire.

9. Film mince biocomposite ou échafaudage tridimensionnel (3D) à utiliser selon la revendication 7 ou 8, dans lequel le sel de magnésium comprend du chlorure de magnésium (MgCl₂), du sulfate de magnésium (MgSO₄) ou du phosphate de magnésium Mg₃(PO₄)₂).

10. Film mince biocomposite ou échafaudage tridimensionnel (3D) produit par le procédé de l'une quelconque des revendications 1 à 6, comprenant une matrice polymère comprenant de la polycaprolactone (PCL), et une charge de magnésium incorporée dans la matrice polymère, dans lequel la charge de magnésium comprend 10 % en poids, 11 % en poids, 12 % en poids, 13 % en poids, 14 % en poids, 15 % en poids, 16 % en poids, 17 % en poids, 18 % en poids, 19 % en poids, ou 20 % en poids, sur la base du poids total du biocomposite, d'un sel de magnésium qui se lixivie ou se dissout au contact d'un fluide corporel pour créer un film mince biocomposite ou un échafaudage tridimensionnel (3D) avec une porosité augmentant graduellement.

11. Film mince biocomposite ou échafaudage tridimensionnel (3D) selon la revendication 10, dans lequel la charge de magnésium ne comprend pas d'alliage de magnésium ou de magnésium élémentaire.

12. Film mince biocomposite ou échafaudage tridimensionnel (3D) selon la revendication 10 ou 11, dans lequel le sel de magnésium comprend du chlorure de magnésium (MgCl₂), du sulfate de magnésium (MgSO₄) ou du phosphate de magnésium (Mg₃(PO₄)₂).
